# EUROPÄISCHE PATENTSCHRIFT

(11) **EP 1 543 803 B1**
(45) Veröffentlichungstag und Bekanntmachung des Hinweises auf die Patenterteilung: **20.12.2006**
(21) Anmeldenummer: 04028205.5
(22) Anmeldetag: 27.11.2004
(51) Int. Cl.: A61F 13/00

(54) **Stütz- oder Druckverband**
Support bandage or compressive bandage
Bande plâtrée ou bande compressive

(30) Priorität: 16.12.2003 DE 10358880
(43) Veröffentlichungstag der Anmeldung: 22.06.2005
(73) Patentinhaber: Germerodt, Hans-Jürgen, 37281 Wanfried (DE)
(72) Erfinder: Germerodt, Hans-Jürgen, 37281 Wanfried (DE)
(74) Vertreter: WALTHER, WALTHER & HINZ Patentanwälte - European Patent Attorneys

(56) Entgegenhaltungen:
- EP-A- 0 556 749
- WO-A-03/047488
- GB-A- 1 331 817
- US-A- 4 525 407
- US-A- 5 209 801
- US-A1- 2002 016 122

## Beschreibung

Die vorliegende Erfindung betrifft einen Stütz- oder Druckverband als Binde, umfassend eine flexible Trägerfolie, wobei auf beiden Seiten der Trägerfolie jeweils eine wellenförmig befestigte Vliesbahn vorgesehen ist, die mit der Trägerfolie verschweißt ist

Aus der GB 1,331,817 ist eine netzartige Trägerschicht bekannt, auf die zu beiden Seiten ein Vlies aufgebracht wird. Die Verbindung der beiden Vliesschichten mit der netzartigen Trägerschicht erfolgt durch Kleben oder Schweißen auf der netzartigen Trägerschicht. In einem nachfolgenden Arbeitsgang erfolgt dann eine Schrumpfung des gesamten Gebildes mit der Folge, dass sich die vliesartige Schicht zu beiden Seiten des netzartigen Trägers mehr oder minder wellt, um einen gewissen Flauscheffekt zu erzielen.

Nach dem Schrumpfvorgang ist davon auszugehen, dass das gesamte Gebilde keine Eigenelastizität mehr aufweist. Insofern ist dieses Material als Material für eine Binde, insbesondere für einen Stütz- oder Druckverband ungeeignet.

Aus der DE 297 24 729 U1 ist ein Film-Vlies-Laminat bekannt, wobei das Vlies auf einem dünngestreckten Film angeordnet ist. Aufgrund der Streckung des Films ist der Film als Trägerschicht nicht flexibel. Eingesetzt werden solche Film-Vlies-Laminate zur Herstellung von Hygieneartikeln.

Die DE-OS 100 34 113 A1 beschreibt die Herstellung eines voluminösen Vliesstoffes. Dieser Vliesstoff wird dadurch voluminös, dass ein Vlies mit Kettfäden durchwirkt und anschließend einer Schrumpfbehandlung unterzogen wird, wodurch sich das Vlies zusammenzieht. Hiernach kann das Vlies zwar gestreckt werden, es geht aber nicht in seine Ausgangslage zurück.

Ein weiterer Stütz- oder Druckverband aus dem Gebrauchsmuster G 90 11 861.8 bekannt, dort auch als medizinischer Schnellverband beschrieben. Der dort dargestellte medizinische Schnellverband dient der Kompression punktierter Arterien bzw. Venen nach invasiver Diagnostik bzw. Therapie, z. B. nach einer Stentimplantation oder auch einer intraaortalen Ballonpulsation. Da für eine derartige Untersuchung die Vene bzw. Arterie im Leistenbereich geöffnet wird, wird ein derartiger Druckverband auch im Leistenbereich eingesetzt. Ein solcher im Leistenbereich eingesetzter Druckverband weist eine im Wesentlichen Y-förmige Gestaltung auf, wie sich dies - wie bereits zu eingangs erwähnt - aus dem zuvor genannten Deutschen Gebrauchsmuster G 90 11 861 ergibt.

Die dort gezeigte Binde besteht zumindest teilweise aus einem elastischen Textil, wobei die Elastizität der Binde eine wesentliche Voraussetzung für die Erstellung eines Druckverbandes ist. Allerdings sind derartige textile Binden relativ teuer. Auf Grund des Preises müssen derartige textile Binden wiederverwendbar sein, d. h. diese Binden werden gewaschen und sterilisiert, was mit einem erhöhten Aufwand verbunden ist.

Aus der EP 0 556 749 A1 ist eine Binde der eingangs genannten Art bekannt. Im Einzelnen ist hierbei vorgesehen, dass eine elastische Trägerfolie beidseitig eine wellenförmig ausgebildete Bahn aufweist, wobei die Bahn mit der elastischen Trägerfolie im Bereich der Wellentäler verbunden ist.

Der Erfindung liegt daher die Aufgabe zu Grunde, eine Binde der eingangs genannten Art bereitzustellen, bei der eine Erhöhung der Stabilität erreicht wird.

Eine solche Binde zeichnet dadurch aus, dass sich im Bereich der Schweißung in der Trägerfolie eine Öffnung ergibt, so dass die Vliesbahnen durch die Trägerfolie hindurch miteinander in Verbindung stehen.

Dem Ziel der verbesserten Atmungsaktivität dient auch, dass im Punkt der Verschweißung der Vliesbahnen zu beiden Seiten der Trägerfolie, die Trägerfolie eine Öffnung aufweist, so dass die zu beiden Seiten der Trägerfolie angeordneten Vliesbahnen miteinander in Verbindung stehen. Es hat sich herausgestellt, dass hierdurch auch eine Erhöhung der Stabilität der Gestaltung der Binde erreicht wird, da die Verformbarkeit bei Dehnung der Binde nicht nur durch die Trägerfolie aufgenommen werden muss, sondern zumindest partiell auch von den Vliesbahnen übernommen wird.

Die flexible Trägerfolie, die beispielsweise aus einem thermoplastischen Elastomer hergestellt ist, hat eine bestimmte Eigenelastizität. Damit die Eigenelastizität nicht durch das darauf befestigte Vlies eingeschränkt ist, ist die wellenförmige Befestigung des Vlieses auf der flexiblen Trägerfolie vorgesehen. Insofern wird die Flexibilität der Trägerfolie im Wesentlichen durch die Größe der Wellen bzw. durch die Eigenelastizität der Trägerfolie begrenzt.

Wie bereits ausgeführt, ist die Vliesbahn mit der Trägerfolie verschweißt ist, wobei vorteilhaft die Trägerfolie perforiert ist, um die Luftdurchlässigkeit und damit den Feuchtigkeitsaustausch zu gewährleisten und somit den Tragekomfort zu erhöhen.

Zur Fixierung der Enden der Binden eines Druckverbandes ist ein Verschluss, insbesondere in Form eines Klettverschlusses vorgesehen.

Anhand der Zeichnung wird die Erfindung nachstehend beispielhaft näher erläutert.

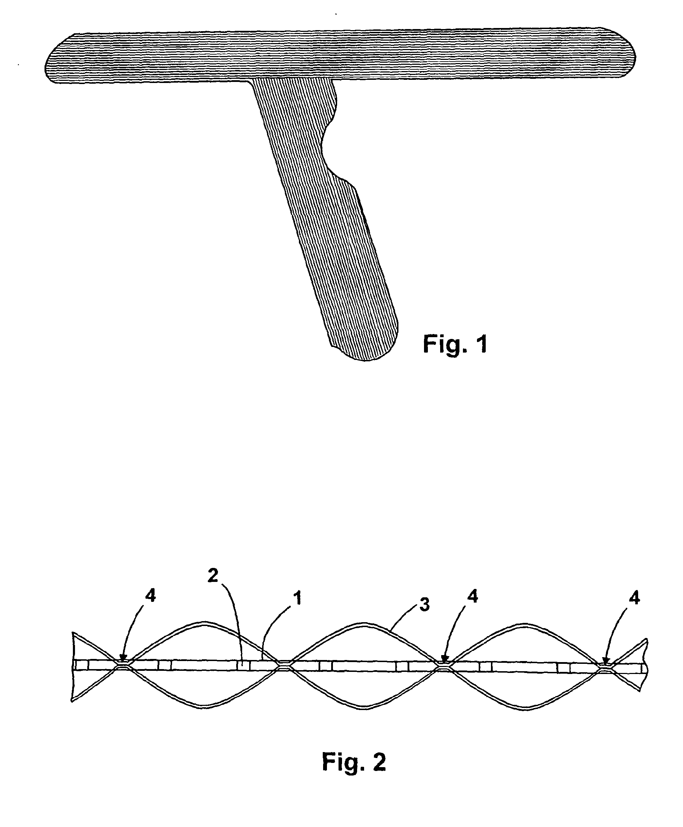

- Figur 1: zeigt schematisch die Gestaltung einer Binde für einen Druckverband;
- Figur 2: zeigt schematisch die Binde im Schnitt.

Die Darstellung eines Stückes einer solchen Binde zeigt eine elastische Trägerfolie 1 mit mehreren Perforationen 2 sowie ein mit der elastischen Trägerfolie verschweißtes Vlies 3, dessen Aufbau wellenförmig ist.

Darüber hinaus ist erkennbar, dass die beiden Vliesbahnen 3 im Bereich ihrer Aufschweißung auf der Trägerfolie durch die Trägerfolie hindurch miteinander in Verbindung stehen (Pfeil 4). Es entsteht hierbei zwischen Folie und den Vliesbahnen eine quasi formschlüssige Verbindung.

## Patentansprüche

1. Stütz- oder Druckverband als Binde, umfassend eine flexible Trägerfolie (1), wobei auf beiden Seiten der Trägerfolie (1) eine wellenförmig befestigte Vliesbahn (3) vorgesehen ist, die mit der Trägerfolie (1) verschweißt ist,
**dadurch gekennzeichnet,**
**dass** sich im Bereich der Schweißung in der Trägerfolie eine Öffnung ergibt, so dass die Vliesbahnen durch die Trägerfolie hindurch miteinander in Verbindung stehen.

2. Binde nach Anspruch 1,
**dadurch gekennzeichnet,**
**dass** die Trägerfolie (1) perforiert ist.

3. Binde nach Anspruch 1,
**dadurch gekennzeichnet,**
**dass** die Binde einen Verschluss, insbesondere einen Klettverschluss aufweist.

## Claims

1. A support or compressive bandage in the form of a swathe, said swathe including a flexible carrier film (1) on either side of which (1) there is fixed, in an undulating manner, a nonwoven web (3) that is welded together with the carrier film (1),
**characterized in**
**that**, in the weld region, an opening is obtained in the carrier film so that the nonwoven webs are connected together through the carrier film.

2. The swathe as set forth in claim 1,
**characterized in**
**that** the carrier film (1) is perforated.

3. The swathe as set forth in claim 1,
**characterized in**
**that** the swathe comprises a fastener, more specifically a Velcro fastener.

## Revendications

1. Pansement compressif ou de contention en forme de bande, du type comprenant une feuille (1) formant support sur chacune des faces de laquelle (1) est prévu un ruban de non-tissé (3) qui est fixé en ondulations et soudé à la feuille (1) formant support,
**caractérisé en ce**
**qu'**une ouverture est obtenue dans la feuille formant support à la hauteur du soudage de sorte que les rubans de non-tissé sont reliés entre eux au travers de la feuille formant support.

2. Bande selon la revendication 1,
**caractérisé en ce**
**que** la feuille (1) formant support est perforée.

3. Bande selon la revendication 1,
**caractérisé en ce**
**que** la bande comporte une fermeture, notamment une fermeture auto-agrippante.
